# EUROPEAN PATENT APPLICATION

(11) **EP 3 485 855 A2**
(43) Date of publication of application: **22.05.2019**
(21) Application number: 18204525.2
(22) Date of filing: 06.11.2018
(51) Int. Cl.: A61F 9/00, B23K 26/046, B23K 26/0622, B23K 26/082, B23K 26/364, G01B 9/02, G02B 21/00, A61F 2/01, B23K 103/00

(54) **LASER PATTERNING APPARATUS OF THREE-DIMENSIONAL OBJECT TO BE PROCESSED AND METHOD THEREOF**

(30) Priority: 15.11.2017 KR 20170152103
(71) Applicant: Advanced Technology Inc., Yeonsu-gu Incheon 21999 (KR)
(72) Inventor: CHOI, Byoung-Chan, 14255 Gwangmyeong-si, Gyeonggi-do (KR); CHOI, Yong Cheol, 22002 Incheon (KR); CHOI, Ho Kyeng, 10403 Goyang-si, Gyeonggi-do (KR); SONG, Young Hun, 21551 Incheon (KR); JUNG, Ki Won, 21331 Incheon (KR); AN, Doo Baeck, 22002 Incheon (KR)
(74) Representative: Vossius & Partner Patentanwälte Rechtsanwälte mbB

(57) **Abstract**

Provided is a laser patterning apparatus of a three-dimensional object to be processed, which includes a laser generation unit (10), a first beam adjustment unit (20) for adjusting the magnitude of a laser beam generated in the laser generation unit, a second beam adjustment unit (30) for adjusting the focal location of z-axis, x-axis, and y-axis of the laser beam via the first beam adjustment unit, and a control unit (70) for controlling the second beam adjustment unit so that laser patterning is performed on a three-dimensional object to be processed. The control unit provides information of a pattern to be processed to one of three-dimensional location formation of a loaded three-dimensional object to be processed and three-dimensional location information included in a three-dimensional shape design file, and performs the alignment through the matching of the three-dimensional location information of the loaded three-dimensional object to be processed and the three-dimensional location information included in the three-dimensional shape design file so that the laser patterning is performed.

## Description

### [Technical Field]

An embodiment of the present disclosure relates to a laser patterning apparatus of a three-dimensional object to be processed and a method thereof.

### [Background Art]

Laser processing means processing an object using a laser beam, and in recent years, the laser processing is also used for the purpose of forming a certain pattern on a processed surface of an object to be processed. A laser patterning apparatus used for such laser processing is a device for forming a predetermined pattern on an object by using a laser.

However, the conventional laser patterning apparatus could not perform patterning on a curved three-dimensional object, and for example, in case of the patterning of the object related to a bio-transplantation body such as an intraocular lens, it was difficult to use it because high precision and error manageability could not be secured.

### [Related Art Document]

### [Patent Document]

(Patent Document 1) Korean Registered Patent No. 10-1243998 (March 08, 2013)

### [Disclosure]

### [Technical Problem]

An object of embodiments of the present disclosure is to provide a laser patterning apparatus for a three-dimensional object to be processed, which affects alignment of cells, the movement direction of cells, adhesion of cells, etc. through a micro or nano pattern in the laser patterning apparatus for a three-dimensional object to be processed.

In addition, another object of the present disclosure is to provide a laser patterning apparatus for a three-dimensional object to be processed, which can produce a nano-sized pattern in a micro scale using a pulsed laser beam.

In addition, still another object of the present disclosure is to provide a laser patterning apparatus for a three-dimensional object to be processed, which can uniformly process a micron-sized line width in a nano scale through a dynamic focusing module capable of adjusting a focal height of the laser beam.

In addition, yet another object of the present disclosure is to provide a laser patterning apparatus for a three-dimensional object to be processed, which can adjust the mobility and adhesiveness of the cell through a micro pattern affecting the alignment of the cell and the movement direction of the cell and a nano pattern affecting the adhesion of the cell.

In addition, still yet another object of the present disclosure is to provide a laser patterning apparatus for a three-dimensional object to be processed, which can acquire surface information of a three-dimensional object to be processed by using one of an optical coherence tomography, a laser interferometer, a confocal microscope, and a two-photon microscope.

An object of embodiments of the present disclosure is to provide a laser patterning method of a three-dimensional object to be processed, which affects the alignment of the cell, the movement direction of the cell, the adhesion of the cell, etc. through a micro or nano pattern in the laser patterning apparatus for the three-dimensional object to be processed.

### [Technical Solution]

Provided is a laser patterning apparatus of a three-dimensional object to be processed, which includes a laser generation unit, a first beam adjustment unit for adjusting the magnitude of a laser beam generated in the laser generation unit, a second beam adjustment unit for adjusting the focal locations of z-axis, x-axis, and y-axis of the laser beam via the first beam adjustment unit, and a control unit for controlling the second beam adjustment unit so that laser patterning is performed on a three-dimensional object to be processed; and the control unit provides information of a pattern to be processed to one of three-dimensional location information of a loaded three-dimensional object to be processed and three-dimensional location information included in a three-dimensional shape design file, and performs the alignment thereof through the matching of the three-dimensional location information of the loaded three-dimensional object to be processed and the three-dimensional location information included in the three-dimensional shape design file so that the laser patterning is performed.

Then, the pattern information can include the shape of the pattern, the width of the pattern, the depth of the pattern, the interval between the patterns, and the wavelength and output, pulse width, scanning speed, spot size, etc. of the laser beam.

In addition, the pattern information can be information that applies one of the three-dimensional location information of the loaded three-dimensional object to be processed and the three-dimensional location information included in the three-dimensional shape design file to three-dimensionally convert the shape of the pattern formed on the plane by the control unit.

In addition, the pattern information can be information that directly generates three-dimensional pattern shape information in one of the three-dimensional shape location information of the loaded three-dimensional object to be processed and the three-dimensional location information included in the three-dimensional shape design file to be converted into three-dimensional information by the control unit.

In addition, the laser generation unit can generate the laser beam of one of nanoseconds, picoseconds, or femtoseconds using a pulsed laser beam source.

In addition, the first beam adjustment unit can adjust the magnitude of the laser beam, and generate the laser beam into a collimated beam.

In addition, the first beam adjustment unit can be a beam expander, and the second beam adjustment unit can include a scan head for adjusting the focal locations of the x-axis and the y-axis and a dynamic focusing module for adjusting the focal location of the z-axis.

In addition, the second beam adjustment unit can include two or more lenses, and can adjust convergence and divergence of the laser beam via the first beam adjustment unit by adjusting the interval between the lenses to adjust the focus of the z-axis of the laser beam.

In addition, the scan head can include a Galvanometer having an x-axis scan mirror and a y-axis scan mirror.

In addition, the laser patterning apparatus of the three-dimensional object to be processed can include a light collection unit for collecting the laser beam on the three-dimensional object to be processed, and the light collection unit can include a telecentric F-theta lens or an F-theta lens.

In addition, the control unit can extract x-axis, y-axis, and z-axis surface shape data of the three-dimensional object to be processed, and can control the dynamic focusing module for adjusting the focal location of the z-axis and the scan head for adjusting the focal locations of the x and y-axes according to the extracted data to form a fine pattern having the pattern width and pattern depth from a micro size to a nano size on the surface of the three-dimensional object to be processed.

In addition, the laser patterning apparatus of the three-dimensional object to be processed can further include a shape recognition unit, and the shape recognition unit can include one of an Optical Coherence Tomography (OCT), a laser interferometer, a confocal microscope, and a two-photon microscope in order to extract surface shape information having x-axis, y-axis, and z-axis of the three-dimensional object to be processed.

In addition, the three-dimensional object to be processed can be a bio-transplantation body, and the laser beam of one of nanoseconds, picoseconds, or femtoseconds can be irradiated to the three-dimensional object to be processed to form a fine pattern.

In addition, the laser patterning apparatus of the three-dimensional object to be processed can include an ultra-precision stage capable of controlling so that the three-dimensional object to be processed is loaded in an effective processing region and an effective focal distance.

Provides is a laser patterning method of a three-dimensional object to be processed, which includes loading a three-dimensional object to be processed in a laser patterning apparatus; acquiring an actually measured three-dimensional location information by measuring the surface of the three-dimensional object to be processed; aligning the three-dimensional object to be processed by matching three-dimensional location information of the object to be processed that has been previously inputted to a control unit on the diagram or the actually measured three-dimensional location information by the control unit; and processing a pattern by irradiating a laser beam on the aligned three-dimensional object to be processed according to information of the pattern.

Then, the pattern information can apply the shape of the pattern formed on the plane to one of the three-dimensional location information of the loaded three-dimensional object to be processed and three-dimensional location information included in the three-dimensional shape design file to be three-dimensionally converted and formed by the control unit.

In addition, the pattern information can be information that directly generates three-dimensional pattern shape information in one of the three-dimensional shape location information of the loaded three-dimensional object to be processed and the three-dimensional location information included in the three-dimensional shape design file to be three-dimensionally converted by the control unit.

In addition, the laser patterning method of the three-dimensional object to be processed can further include managing quality for inspecting the shape of the pattern, the width of the pattern, the depth of the pattern, the interval between the patterns, and the wavelength and output, pulse width, scanning speed, and spot size of the laser beam with respect to the object to be processed that the pattern has been processed. In addition, it can further include generating an analysis report through information inspected in the managing the quality.

### [Advantageous Effects]

According to the embodiment of the present disclosure, it is possible to provide the laser patterning apparatus for the three-dimensional object to be processed, which affects the alignment of the cell, the movement direction of the cell, the adhesion of the cell, etc. through a micro or nano pattern in the laser patterning apparatus for the three-dimensional object to be processed.

In addition, it is possible to provide the laser patterning apparatus for the three-dimensional object to be processed, which can produce the nano-sized pattern in the micro scale by using the pulsed laser beam.

In addition, it is possible to uniformly process a micron-sized line width in the nano scale through the dynamic focusing module capable of adjusting the focal height of the laser beam.

In addition, it is possible to adjust the mobility and adhesiveness of the cell through the micro pattern affecting the alignment of the cell and the movement direction of the cell and the nano pattern affecting the adhesion of the cell.

In addition, it is possible to adjust the behavior and function of the cell such as the adhesion, movement, and differentiation of the cell according to the size of the pattern, and using the above, it is possible to provide the function of the pattern such as the infection prevention, antibacterial, prevention of the vascular restenosis and late thrombus formation of a stent, facilitation of the bone formation and bone adhesion of dental and orthopedic implants, and prevention of posterior cataract of the intraocular lens, thus manufacturing a bio-transplantation-type functional medical device.

In addition, in order to strengthen the biocompatibility of the implantable medical device, it is possible to form the micro-nano pattern on the surface of the medical device having the three-dimensional complex shape.

In addition, the implantable medical device can be a cardiovascular or non-vascular stent, a dental or orthopedic implant, etc.

In addition, it is possible to acquire the surface information of the three-dimensional object to be processed by including one of an Optical Coherence Tomography (OCT), a laser interferometer, a confocal microscope, and a two-photon microscope.

According to embodiments of the present disclosure, in the laser patterning apparatus for the three-dimensional object to be processed, it is possible to provide a laser patterning method of the three-dimensional object to be processed, which affects the alignment of the cell, the movement direction of the cell, the adhesion of the cell, etc. through a micro or nano pattern.

### [Description of Drawings]

FIG. 1 is a diagram illustrating a beam path of a laser pattering of a three-dimensional object to be processed in accordance with an embodiment of the present disclosure.
FIG. 2 is a diagram illustrating a beam path of the laser patterning of the three-dimensional object to be processed in accordance with an embodiment of the present disclosure.
FIG. 3 is a diagram illustrating the range of beam of the laser patterning in accordance with an embodiment of the present disclosure.
FIG. 4 is a flowchart for explaining a flow for processing an object to be processed in accordance with an embodiment of the present disclosure.
FIG. 5 is a flowchart for specifically explaining the flow for processing the object to be processed in accordance with an embodiment of the present disclosure.
FIG. 6 is a flowchart for specifically explaining the flow for processing the object to be processed in accordance with an embodiment of the present disclosure.
FIG. 7 is a flowchart for specifically explaining the flow for processing the object to be processed in accordance with an embodiment of the present disclosure.
FIG. 8 is a diagram illustrating a procedure that location information of the object to be processed in accordance with an embodiment of the present disclosure is data-converted.
FIG. 9 is a diagram illustrating the object to be processed in accordance with an embodiment of the present disclosure.
FIG. 10 is a diagram illustrating the object to be processed in accordance with another embodiment of the present disclosure.
FIGS. 11 and 12 are diagrams illustrating patterns in accordance with an embodiment of the present disclosure.
FIG. 13 is a diagram illustrating an alignment procedure of the object to be processed in accordance with an embodiment of the present disclosure.
FIG. 14 is a diagram illustrating an intraocular lens that the patterning in accordance with an embodiment of the present disclosure is completed.

### [Best Mode]

Hereinafter, specific embodiments of the present disclosure will be described with reference to the drawings. However, it is merely an example, and the present disclosure is not limited thereto.

In the following description of the present disclosure, a detailed description of known technology related to the present disclosure will be omitted when it is determined to unnecessarily obscure the subject matter of the present disclosure. Then, the following terms are defined in consideration of the functions of the present disclosure, and can be changed according to the intention or custom of the user, the operator, etc. Accordingly, the definition should be based on the contents throughout this specification.

The technical spirit of the present disclosure is determined by the claims, and the following embodiments are merely a means for effectively explaining the technical spirit of the present disclosure to a person having ordinary skill in the art to which the present disclosure pertains.

The laser patterning apparatus for the three-dimensional object to be processed in accordance with the present disclosure can be used for the bio-transplantation processing of a bio-transplantation body, for example, an intraocular lens, a dental implant, or an orthopedic implant. Meanwhile, at least one of a micro pattern and a nano pattern, which affects the alignment and movement direction of the cell in the bio-transplantation body, by including a laser generation unit and a beam adjustment unit, can be patterned. Herein, as an example of the object to be processed, the object to be processed that can be transplanted or placed in a living body is described as an example, but it is natural that the present disclosure is not limited thereto, and can include an object to be processed by including a pattern that can be used in the human body or with the human body.

Hereinafter, the laser patterning apparatus of the three-dimensional object to be processed and the method thereof will be described separately, but it is natural that the description in the apparatus will be omitted, and the specific steps described only in the description of the method will be performed through the apparatus in accordance with the embodiment of the present disclosure.

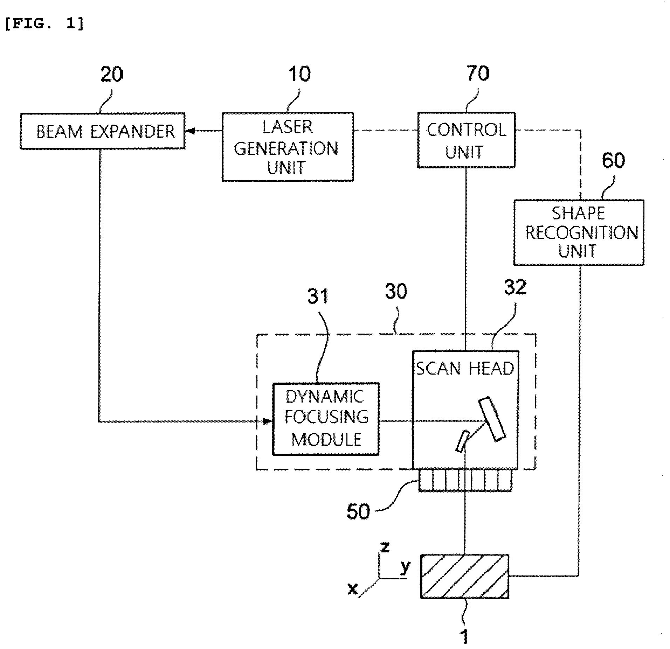

FIG. 1 is a diagram illustrating the laser beam path of a laser patterning apparatus of a three-dimensional object to be processed in accordance with an embodiment of the present disclosure.

Referring to FIG. 1, a laser patterning apparatus for a three-dimensional object to be processed in accordance with the present disclosure can include a laser generation unit 10, a beam expander 20, a beam adjustment unit 30, a light collection unit 50, a shape recognition unit 60, and a control unit 70 in order to perform fine patterning on a three-dimensional object to be processed 1.

The laser generation unit 10 can generate a laser beam for patterning. Specifically, the laser generation unit 10 can use a pulsed laser source. Accordingly, the laser generation unit 10 can generate one laser beam of nanoseconds, picoseconds, or femtoseconds. Among them, for example, the femtosecond laser beam can be an ultra high-frequency laser having a pulse duration time of 1 to 1000 femtoseconds. Specifically, the laser generation unit 10 can generate a pulsed laser beam having a pulse duration time within a femtosecond range. Herein, a pulse repetition rate can be in the range of two digit kHz to the maximum three digit kHz, or can be in the range of MHz. The wavelength of the laser beam can be all of the laser wavelengths located from the infrared region to the ultraviolet region. For example, an infrared wavelength, a green wavelength, an ultraviolet wavelength, etc. can be included therein.

The laser patterning apparatus for the three-dimensional object to be processed in accordance with an embodiment of the present disclosure can pattern a pattern having a width and depth in a unit of nanometer on the three-dimensional object to be processed 1 in a micro-scale. By converting and using the laser wavelength according to the size of the designed pattern, the sizes of various patterns can be realized. For example, when the three-dimensional object to be processed is an intraocular lens, which is one of the bio-transplantation bodies, a laser beam can be at once irradiated to the entire surface of the object to be processed having a diameter of 10mm or more (preferably, 12mm) to generate a pattern from several micro units to a nano unit. In addition, the laser patterning apparatus for the three-dimensional object to be processed in accordance with an embodiment of the present disclosure can use it by converting the wavelength of the laser to have a short wavelength in the ultraviolet region, thus overcoming the diffraction limit of the light collection unit 50 and implementing the pattern of the nano unit.

The laser beam generated in the laser generation unit 10 can be a pulsed femtosecond laser beam, and can pass through the beam expander 20 and the beam adjustment unit 30.

The beam expander 20 can adjust the magnitude of the laser beam generated in the laser generation unit 10. Specifically, the beam expander 20 can enlarge or reduce the laser beam. In addition, the beam expander 20 can generate a laser beam as a collimated beam with little dispersion or concentration. Accordingly, the laser beam generated in the laser generation unit 10 can be generated as a collimated beam while being enlarged or reduced in its size via the beam expander 20. The magnitude of the laser beam changed from the beam expander 20 can be the magnitude of the laser beam incident on the lens at the last stage of the laser patterning apparatus of the three-dimensional object to be processed in accordance with an embodiment of the present disclosure. The beam expander 20 can change the diameter of the laser beam generated in the laser generation unit 10, and output the changed laser beam. The beam expander 20 can be manually or automatically adjustable.

In addition, various optical elements such as a beam attenuator, a polarizing plate, a half wave plate, a splitter, a filter, and a shutter can be further located.

The beam adjustment unit 30 can adjust the focal height and focal location of the laser beam that can be irradiated to the three-dimensional object to be processed. The beam adjustment unit 30 can include a dynamic focusing module 31 and a scan head 32. The dynamic focusing module 31 of the beam adjustment unit 30 can adjust the focal height of the laser beam, and the scan head 32 can adjust the focal location of the laser beam along the 3-dimensional object to be processed.

The dynamic focusing module 31 can adjust the focal location of the laser beam passing through the light collection unit 50 according to the three-dimensional processing data of the three-dimensional object to be processed. Specifically, the dynamic focusing module 31 can include two or more lenses. By adjusting the interval between the respective lenses, the focus of the laser beam passing through the light collection unit 50 can be adjusted by adjusting the divergence and convergence of the laser beam passing through the dynamic focusing module 31.

That is, the dynamic focusing module 31 can adjust the focal height of the laser beam passing through the beam expander 20, that is, the z-axis location of the focus. The dynamic focusing module 31 can adjust the z-axis location of the laser beam, that is, the focal height of the laser beam, by adjusting the convergence and divergence of the laser beam passing through the beam expander 20.

The dynamic focusing module 31 can irradiate it by adjusting the distance of the laser beam emitted to the scan head 32 by driving a motor (not illustrated) that reciprocates horizontally. For example, when the motor is horizontally reciprocated and the dynamic focusing module 31 moves to the left side thereof, the focus of the laser beam moves away from the three-dimensional object to be processed 1, such that it can move to the upside of the floor of FIG. 1 on the z-axis thereof. Accordingly, the height of the laser beam can be shortened. On the contrary, when the dynamic focusing module 31 is moved to the right side thereof, the laser beam approaches the three-dimensional object to be processed 1, such that the focus of the laser beam can also move to the downside of the floor of FIG. 1 on the z-axis thereof. Accordingly, the height of the laser beam can be lengthened. Accordingly, the focal location of the laser beam incident on the three-dimensional object to be processed 1 can be controlled in the z-axis direction thereof.

It is possible to perform patterning along the height of the surface of the three-dimensional shape of the three-dimensional object to be processed 1 through the dynamic focusing module 31. For example, since the intraocular lens, which is one of the bio-transplantation bodies, has a curved shape, the location at which the pattern is to be patterned by the laser beam can be different in height (i.e., a Z axis) according to x-axis and y-axis thereof, respectively. Adjustment of the location of the focus of the laser beam through the dynamic focusing module 31 on the z-axis thereof can implement uniform patterning corresponding to different z-axis locations for each of the x axis and y axis coordinates. In addition, patterning can be performed in the width from a nano size to a micro size. In addition, the shape of the pattern can be a point, a dotted line, a line, a poly line, an arc, a polygon, etc. The dynamic focusing module 31 can move an internal optical system or move each lens included in the optical system. Accordingly, the height of the laser beam can be controlled at high speed in real time, such that it is possible to enhance the uniformity of the line width on the surface of the three-dimensional object to be processed and to enhance the productivity thereof.

The x-axis and y-axis focal locations of the laser beam whose z-axis focal location has been adjusted by the dynamic focusing module 31 can be adjusted by the scan head 32.

The scan head 32 can adjust the focal locations of the x-axis and y-axis of the three-dimensional object to be processed 1. The scan head 32 includes an x-axis scan mirror (not illustrated) and a y-axis scan mirror (not illustrated) to perform two-dimensional scanning. The laser beam whose z-axis focal location has been adjusted by the dynamic focusing module 31 can be finely controlled in the x-axis and y-axis directions along the curved surface of the three-dimensional object to be processed 1 through the x-axis scan mirror and the y-axis scan mirror.

The x-axis scan mirror and y-axis scan mirror of the scan head 32 can reflect the laser beam in the direction for patterning the laser beam to irradiate the laser beam to a desired location of the three-dimensional object to be processed 1. The x-axis scan mirror and the y-axis scan mirror are composed of a pair of scan mirrors in the form of a Galvanometer, each of which deflects the laser beam in one direction of the axes crossing each x-y plane.

Accordingly, as described above, the beam adjustment unit 30 can adjust the focal height and focal location of the laser beam. The laser beam can be enlarged or reduced in its size via the beam expander 20, and can be refracted in the direction that is generated as a collimate beam and controlled. The z-axis focal location of the laser beam having passed through the beam expander 20 can be adjusted by the dynamic focusing module 31, and the x and y coordinates can be adjusted by the scan head 32 to adjust the focal location of the laser beam to be corresponded to the three-dimensional object to be processed 1.

The light collection unit 50 for collecting the femtosecond laser beam having passed through the dynamic focusing module 31 and the scan head 32 to the three-dimensional object to be processed 1 can be located on the lower portion of the beam adjustment unit 30.

The light collection unit 50 can collect the laser beam. The light collection unit 50 can collect the laser beam having passed through the beam adjustment unit 30 to irradiate the laser beam to the three-dimensional object to be processed 1. The light collection unit 50 can include a telecentric F-theta lens or an F-theta lens. As a result, a fine pattern of a micro-sized or nano-sized unit can be processed.

Through these configurations, at least one of various parameters such as the irradiation location and focal distance of the laser beam, and the pulse waveform, irradiation time, scanning speed, divergence characteristic, and astigmatism of the laser beam to be output can be adjusted.

The shape recognition unit 60 can recognize the shape of the three-dimensional object to be processed 1. The shape recognition unit 60 can be located in a space different from the laser beam path as illustrated in FIG. 1. In addition, the shape recognition unit 60 can be also located in a path through which the laser beam is delivered between the dynamic focusing module 31 and the scan head 32. The shape recognition unit 60 can recognize the surface of the three-dimensional curved shape of the three-dimensional object to be processed 1 through the interference phenomenon of light and can display it as a diagram. The shape information of the three-dimensional object to be processed 1 can be acquired through the interferometer using the refractive index to be transmitted to the control unit 70. Specifically, there has been a problem in that since the transparent curved three-dimensional object to be processed 1 is transparent, it is difficult to recognize the height and surface thereof. Accordingly, it is possible to acquire the three-dimensional information of the surface of the object to be processed 1 through the interferometer using the refractive index, and to match the acquired information with the diagram inputted to the control unit 70, thus finding a specific point (e.g., a vertex of the curved shape) of the three-dimensional object to be processed 1. Accordingly, it is possible to confirm the location of the patterning processing by irradiating the laser beam. Through the shape recognition unit 60, patterning can be performed flexibly with respect to various surface structures.

In addition, the shape recognition unit 60 can recognize the shape of the three-dimensional object to be processed 1 by including the optical coherence tomography (OCT). For example, the surface of the three-dimensional object to be processed 1 having a transparent curved shape can be scanned three-dimensionally using a laser beam as a light source for inspection to measure the coordinates of the three-dimensional surface shape, and laser patterning can be performed on the surface of the three-dimensional object to be processed based on the data. Herein, it is natural that the laser for laser patterning can be one of nanoseconds, picoseconds, or femtoseconds laser. For example, the wavelength of the laser beam can be greater than 100nm and equal to or less than 10000nm, and the repetition rate thereof can be a laser beam that is from 1Hz to several hundred GHz.

In addition, it is natural that the shape recognition unit 60 is not limited thereto, and can recognize the shape of the three-dimensional object to be processed 1 by including a confocal microscope or a two-photon microscope. Herein, the confocal microscope is a microscope using a confocal principle, and the shape recognition unit 60 including the same removes light from the laser beam that does not fit the focus of the three-dimensional object to be processed 1, and uses only the light that matches the focus of the three-dimensional object to be processed 1, thus recognizing the shape of the three-dimensional object to be processed 1. In addition, the shape recognition unit 60 can recognize the shape of the three-dimensional object to be processed 1 by including the two-photon microscope using the two-photon absorption phenomenon.

The control unit 70 can input the designed three-dimensional pattern data to extract the focal location data of the x-axis, y-axis, and z-axis thereof in order to perform patterning on the surface of the three-dimensional object to be processed having a curved surface. Based on this data, the two-dimensional focal location data of the x-axis and y-axis thereof can be controlled by the scan head 32. In addition, the focal location data of the z-axis can be controlled by the dynamic focusing module 32 to control the three-dimensional pattern data in real time. Accordingly, it is possible to produce a fine pattern having the pattern width and pattern depth from a micro unit to a nano unit on the surface of an intraocular lens.

Accordingly, the laser patterning device of the three-dimensional object to be processed 1 in accordance with an embodiment of the present disclosure can extract the three-dimensional surface shape information of the bio-transplantation body, for example, the intraocular lens having a transparent curved surface by including one of a laser interferometer, a confocal microscope, and a two-photon microscope. Based on the above, a laser beam can be irradiated on the surface of the three-dimensional object to be processed to produce a pattern width and pattern depth from a micro size to a nano size. Of course, the laser for laser patterning can be one of nanoseconds, picoseconds, or femtosecond lasers.

As a result, when the laser patterning is performed on the surface of the three-dimensional object to be processed 1, it is possible to overcome a defect in a pattern location to be processed, a defect in a pattern not processed, a defect in a pattern broken, a defect in a pattern overlapped, a defect in product surface scratch, etc.

The laser patterning apparatus of the three-dimensional object to be processed in accordance with an embodiment of the present disclosure can further include an ultra-precision stage (not illustrated). There is a possibility that when the three-dimensional object to be processed 1 is mounted on the stage for processing, deformation that deviates from the effective focal distance of the optical system is likely to occur. Accordingly, the three-dimensional object to be processed 1 can be controlled to be located within the effective regions to be processed and effective focal distances of the dynamic focusing module 31 and the scan head 32 according to the combination of a large number of axes in the coordinate system defined by the nano-scale ultra-precision stage.

Meanwhile, although not illustrated in the drawings, for example, the three-dimensional object to be processed 1 can be an intraocular lens. Hereinafter, the intraocular lens will be described as an example thereof when the three-dimensional object to be processed will be described as an example, but it is natural that the present disclosure is not limited thereto, and can include any object that can be placed in or inserted into a human body such as an implant, a stent, etc. and an object that can be used together with the human body.

For example, the intraocular lens can include an optic part in the central region thereof and a haptic part in the peripheral region thereof. The laser patterning apparatus for the three-dimensional object to be processed in accordance with an embodiment of the present disclosure can perform fine patterning by irradiating a femtosecond laser beam onto the haptic part of the intraocular lens. It is possible to form patterns of various shapes in a micro or nano unit in the haptic part so that the cell can be aligned to have the directionality, moved, and adhered thereto.

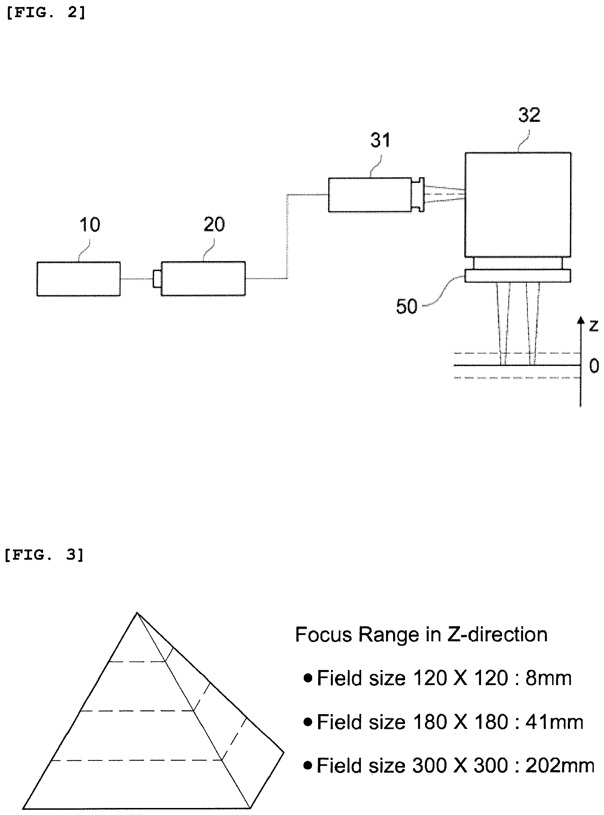

FIGS. 2 and 3 are diagrams illustrating the beam paths of the laser patterning of the three-dimensional object to be processed in accordance with an embodiment of the present disclosure.

Referring to FIG. 2, the laser beam generated in the laser generation unit 10 passes through the beam expander 20 to be delivered to the beam adjustment unit 30. The z-axis of the laser beam delivered to the beam adjustment unit 30 can be adjusted by the dynamic focusing module 31, and the x-axis and y-axis thereof can be adjusted by the scan head 32.

Referring to FIG. 3, a laser beam whose x-axis, y-axis, and z-axis are adjusted by the dynamic focusing module 31 and the scan head 32 can be irradiated on the three-dimensional object to be processed 1.

For example, the magnitudes of the laser beams irradiated to the x-axis and y-axis fields (i.e., the image-forming surfaces) having different heights of the three-dimensional object to be processed 1 can be the same. The sizes of the x-axis and y-axis that can be scanned are determined according to the specification of the focusing lens of the light collection unit 50, and accordingly, the range of the z-axis thereof can be determined. When the scanning field size of the x-axis and the y-axis is 120mm x 120mm, the focus range in the Z-direction can be 8mm. In addition, when the scanning field size of the x-axis and the y-axis is 180mm x 180mm, the focus range in the Z-direction can be 41mm, and when the scanning field size of the x-axis and the y-axis is 300mm x 300mm, the focus range in the Z-direction can be 202mm. That is, the dynamic focusing module 31 can adjust the z-axis corresponding to the coordinate value of the x-axis and the y-axis adjusted by the scan head 32 according to the three-dimensional pattern data of the three-dimensional object to be processed 1 that is inputted to the control unit 70.

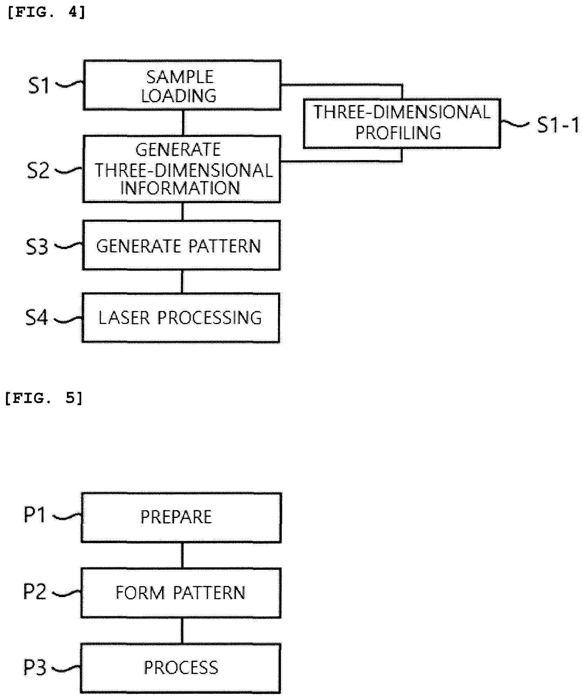

FIGS. 4 and 5 are flowcharts for explaining flows of processing the object to be processed in accordance with an embodiment of the present disclosure.

Referring to FIG. 4, a method for processing the object to be processed can include loading the object to be processed S1, generating three-dimensional information of the object to be processed S2, generating a pattern to be processed on the object to be processed S3, and processing the object to be processed through a laser S4. In the above procedure, a three-dimensional profiling S1-1 can be performed between the loading the object to be processed S1 and the generating the three-dimensional information of the object to be processed S2.

First, a processing method that does not include the three-dimensional profiling S1-1 will be described in detail, and it is possible to load a transparent object to be processed, and to input a predetermined shape design file of the object to be processed into the apparatus. Herein, the shape design file includes information on the object to be processed at the time of manufacturing the transparent object to be processed, and can include three-dimensional location information. Accordingly, location information of the line and plane in the vertical and horizontal directions can be included therein. That is, the object to be processed that is manufactured according to the location information included in the shape design file can coincide with the location information.

Meanwhile, a pattern to be processed can be inputted to the object to be processed based on the location information. That is, a pattern to be processed by a laser later can be recorded as a value on the location information.

Then, the location and alignment state of the object to be processed already loaded can be inspected. The inspection is a procedure for confirming that the location information included in the shape design file is matched therewith, and the inspected object to be processed can perform the inspection procedure with the location information previously inputted to match the location information of the confirmed object to be processed. That is, the loaded object to be processed or scan head 32 can be moved and aligned so that the inspected location information of the object to be processed and the location information included in the shape design file are superimposed (matched) with each other.

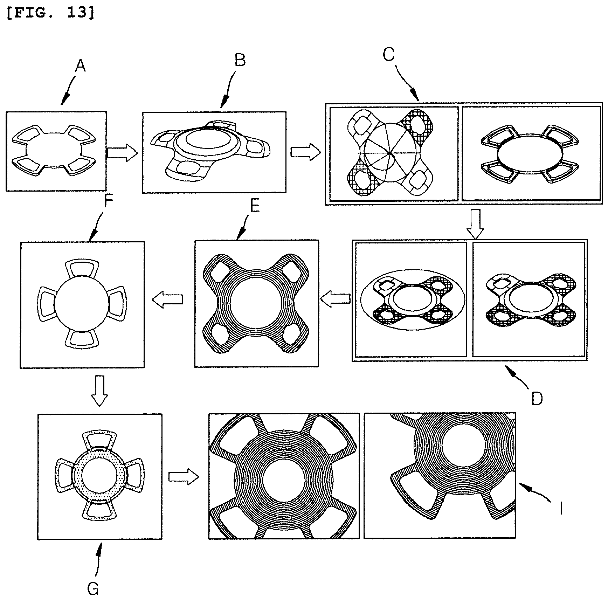

Referring to FIG. 13 with respect to the above alignment, the alignment state can be implemented through matching of the three-dimensional pattern data A and a measured image B of the object to be processed. Herein, the measured image B of the object to be processed can be three-dimensional. Accordingly, it can be matched with the three-dimensional pattern data A, and the alignment of the object to be processed 1 actually loaded can be controlled by the matched result. In order to confirm the alignment state by the matching C of each data and to correct the alignment state, rotation or tilting of a loading part (not illustrated) in which the object to be processed 1 is loaded can be performed, and the object to be processed 1 can be moved from the location before alignment to be aligned.

Then, the input pattern can be processed through the laser on the surface of the aligned object to be processed. After the processing, the quality inspection of the patterned object to be processed can be selectively performed. The quality inspection can include confirming whether or not the pattern inputted based on the location information included in the shape design file is formed to be matched with the object to be processed through the laser, and can be a procedure for inspecting whether or not the size of the pattern processed through the laser, the surface roughness of the surface processed through the laser, etc. meet a predetermined criterion. Of course, the degree of matching of the pattern can be predetermined by a person skilled in the art, and for example, the width between uneven parts, the depth and width of the uneven part, etc. formed by the pattern processing can become a criterion for determining the degree of matching.

Meanwhile, as described above, the method for processing the object to be processed further including the profiling S1-1 can perform the three-dimensional profiling S1-1 between the loading the object to be processed S1 and the generating the three-dimensional information of the object to be processed S2.

Accordingly, the same description as the above-described procedure is omitted, and the description of the profiling S1-1 will be described in detail. After the three-dimensional shape design file of the object to be processed is inputted, the three-dimensional profile of the already-loaded object to be processed can be inspected. Herein, the three-dimensional profile means that the location information confirmed to confirm the location information in the vertical and horizontal directions of the object to be processed is inputted to the control unit 70, and the diagram including the three-dimensional location information can be generated as data. Based on the data, a pattern to be processed through the laser on the object to be processed can be additionally generated.

The pattern generated based on the data can be processed by the laser after the location information included in the three-dimensional shape design file is matched with the actually measured location information of the object to be processed to be aligned.

The above-mentioned two processing methods are to process by matching the actually measured three-dimensional location information of the object to be processed with the location information included in the shape design file to align it, and include a difference in that the object providing information of an initial pattern is the location information included in the shape design file or the location information of the actually measured object to be processed. This can be selectively determined, and pattern information can be provided to a highly reliable object according to the processing environment, and then matching between two location information for alignment can be performed.

In addition, although it has been described that the calculation required to perform the above-described series of procedures can be performed through the control unit 70, it can further include a separate control unit to process the calculation. The calculation is a calculation that is required for correcting and adjusting the processing location and tilt of the object to be processed based on the input information such as the location, alignment state, tilt, three-dimensional outline information, etc. of the object to be processed.

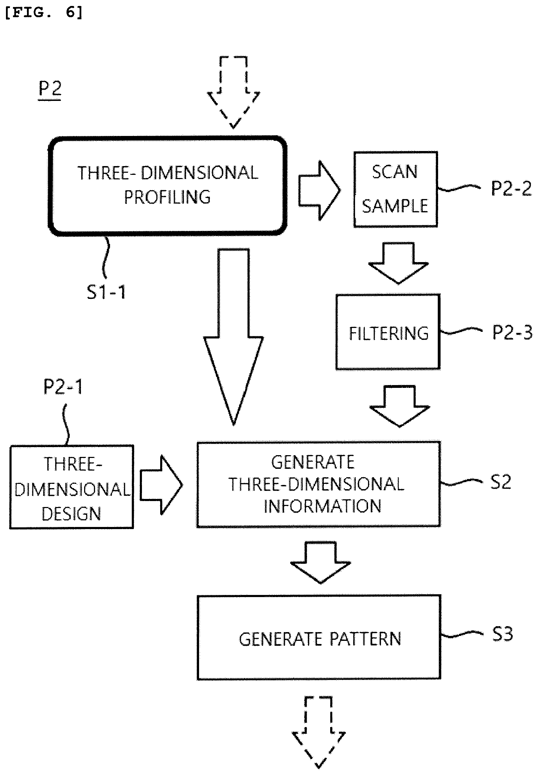

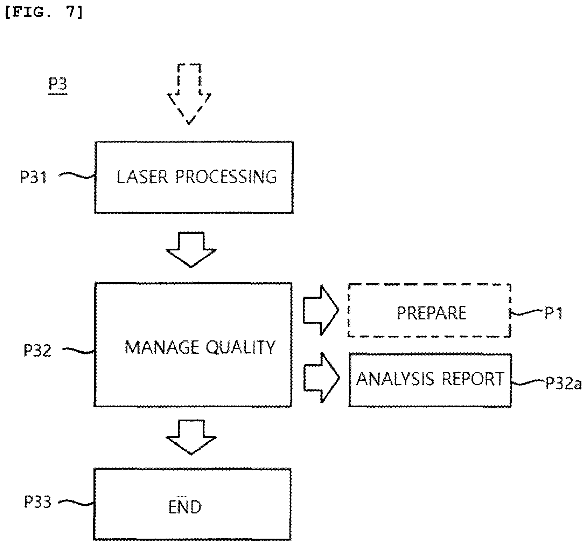

Referring to FIG. 5, the method for processing the three-dimensional object to be processed of the present disclosure includes each step of FIG. 4, and can be more simply divided into preparing P1, forming a pattern P2, and processing P3. When the loading the object to be processed corresponds to the preparing P1, the forming the pattern P2 and the processing P3 can include various details. These detailed steps will be described later with reference to FIGS. 6 and 7.

FIGS. 6 and 7 are flowcharts for specifically explaining flows for processing the object to be processed in accordance with an embodiment of the present disclosure.

First, referring to FIG. 6, the details included in the forming the pattern P2 will be described. The three-dimensional profiling S1-1 for the loaded object to be processed can be performed. The three-dimensional profiling S1-1 is to confirm the three-dimensional location information of the object to be processed, and the three-dimensional location information can be data that is matched with the location information included in the previously inputted three-dimensional shape design file later.

The pattern information can be inputted to at least one of the three-dimensional location information of the confirmed object to be processed or the location information included in the previously input three-dimensional shape design file.

When the manufacturing process is sequentially performed according to "a first path" in which the pattern information is inputted to the actually measured three-dimensional location information of the object to be processed, the surface of the sample that is the object to be processed is scanned P2-2; the pattern information is inputted to the location information which is the surface of the object to be processed having a curved surface through a filtering P2-3 by software, etc.; and the matching between the location information and the location information included in the previously inputted three-dimensional shape design file can be performed. As described above, the object to be processed and the scan head 32 can be aligned after the matching S2 between the respective location information is performed. The pattern S3 generated before or after the alignment can be applied to be set to the state immediately before the processing.

The above-described series of procedures are described by a sequence through an example, and the sequence between the details in the forming the pattern P2 process can be freely located. That is, in addition to the above-described sequence, the pattern information can be inputted to the location information before and after the matching.

Unlike the above-described "the first path," "a second path" is as follows.

The three-dimensional profiling S1-1 can be performed on the object to be processed to obtain the location information of the object to be processed, and the location information and the location information included in the previously inputted three-dimensional shape design file can be matched. Herein, the pattern information processed by the laser on the object to be processed can be inputted before or after the matching based on the location information included in the three-dimensional shape design file. The laser processing can be performed according to the pattern information inputted herein, and the actually measured location information of the object to be processed and the location information included in the three-dimensional shape design file can be matched with each other to be aligned before the laser processing is performed. This alignment means that it is performed up to the immediately preceding step thereof so that the laser processing can be performed.

The above-described series of procedures are also described by a sequence through an example, and the sequence between the details in the forming the pattern P2 process can be freely located.

Referring to FIG. 7, the details of the processing P3 can be described. As a step that can be performed after the forming the pattern P2 described with reference to FIGS. 5 and 6, the laser processing P31 can be performed on the object to be processed in the aligned state. Herein, the laser processing P31 means that the object to be processed is processed by the laser according to the pattern information. In this time, the control unit 70 can include laser processing parameter information such as a wavelength, output, pulse width, spot size, etc. of the laser, and information on the width of the pattern and the interval between the patterns so that the laser can be irradiated thereto.

After the laser treatment P31 is performed, managing quality P32 for checking the surface of the processed object to be processed, that is, the state of the pattern, can be performed. The managing the quality P32 can be performed by the above-described non-contact method by the scan head 32, etc. When the managing the quality P32 is completed, the patterning of the patterning device can be ended P33. Of course, the ending P33 means the end of one cycle of patterning, such that it is possible to unload the object to be processed that the patterning is completed, and to load a new object to be processed.

Meanwhile, it can further include an additional step. The additional step can be, for example, generating an analysis report P32a. The generating the analysis report P32a means storing information related to quality while performing the managing the quality P32, and also outputting the stored information. It is possible to accumulate such quality management information to maintain the uniform patterning quality of the object to be processed.

As an example of the present disclosure, the three-dimensional object to be processed can be an intraocular lens, and the intraocular lens can include an optic part and a haptic part. The three-dimensional object to be processed can include a curved surface of the surfaces, and for example, in the intraocular lens, the surface of the optic part can be a curved surface. In addition, the processing of the pattern can be formed at least on the optic part, and preferably, can be formed in the vicinity of the edge of the optic part that is circular. More accurately, when the object to be processed is the intraocular lens, it is possible to prevent the pattern from being located on a path that the light is inputted to a cornea.

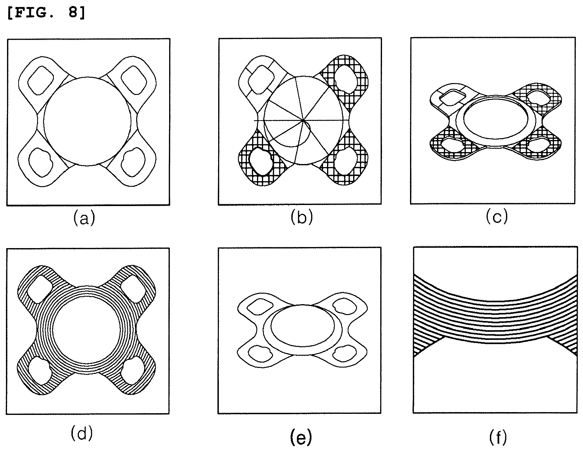

FIG. 8 is a diagram illustrating a procedure of data-converting location information of the object to be processed in accordance with an embodiment of the present disclosure.

Referring to FIG. 8, the filtering through the software, etc. described above with reference to FIG. 6 is illustrated, and FIG. 8(a) can become a three-dimensional design diagram of the object to be processed or a three-dimensional diagram based on location information of the object to be processed obtained by the three-dimensional profiling. That is, the location information can be acquired by coordinating the diagram illustrated through the location information on the plane side thereof (FIG. 8(b)), and the curved coordinates can be acquired in the vertical direction in order to acquire the location in the vertical direction from the plane (FIG. 8(c)). Accordingly, the three-dimensional location information of the object to be processed can be coordinated.

Referring to FIG. 8(d), when a virtual pattern, that is, a pattern diagram to be processed is provided on the plane in the acquired three-dimensional location information and the virtual pattern diagram provided on the plane is applied on the three-dimensional location information, the pattern diagram provided on the plane thereof can be converted into a pattern having three-dimensional location information (FIG. 8(e)). The pattern having the converted three-dimensional location information can be processed on the surface of the object to be processed through the laser delivering a signal from the control unit to be irradiated (FIG. 8(f)).

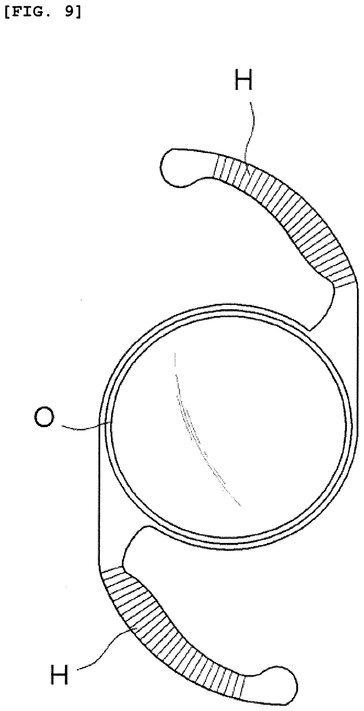

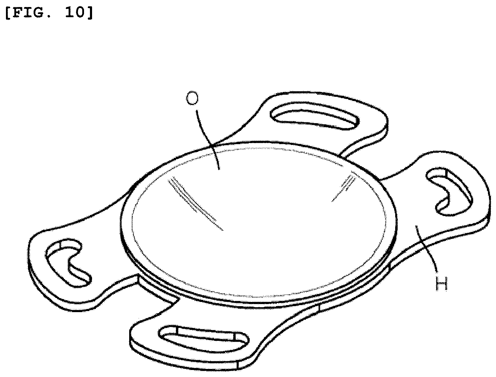

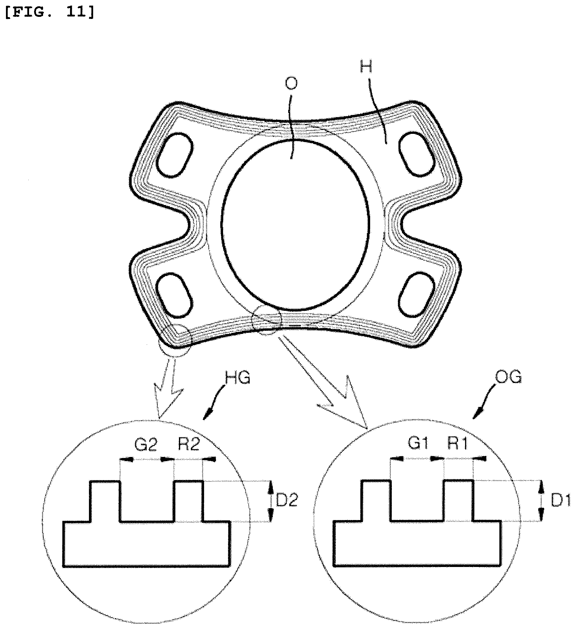

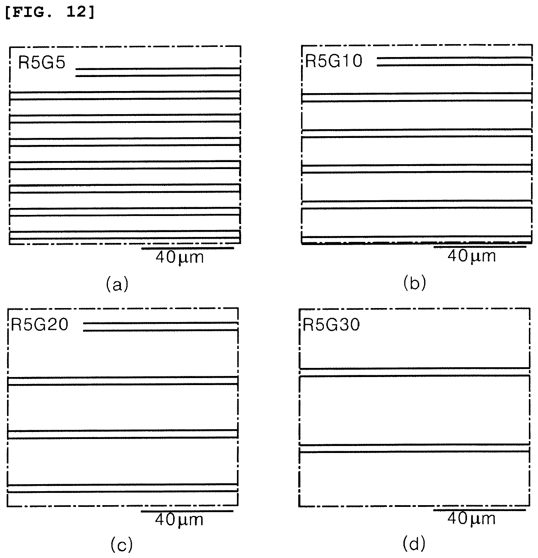

FIG. 9 is a diagram illustrating an exemplary three-dimensional object to be processed in accordance with an embodiment of the present disclosure, FIG. 10 is a diagram illustrating a three-dimensional object to be processed in accordance with another embodiment of the present disclosure, and FIGS. 11 and 12 are diagrams illustrating patterns in accordance with an embodiment of the present disclosure. Hereinafter, the case where the three-dimensional object to be processed is an intraocular lens is mainly described as an example. However, it is natural that it is not limited thereto, and the three-dimensional object to be processed can include any three-dimensional object to be processed that can be placed in or inserted into a human body such as an implant, a stent, etc., and a three-dimensional object to be processed that can be used together with the human body.

First, referring to FIGS. 9 and 10 among FIGS. 9 to 12, the object to be processed 1 can be provided that a pair of haptic parts H extended from one optic part O are formed, or two pairs of haptic parts H are formed from one optic part O. In the two embodiments, the haptic part H and the optic part O can have a pattern for each region, or a pattern can be also formed across the two regions. In the following example, a case where a pattern is formed across the two regions will be described as an embodiment, but it can be identically applied in that the information (shape, depth, interval, width, etc.) of the pattern formed on the characteristic part, that is, the optic part O and the haptic part H can be different.

In describing an example below with reference to FIGS. 11 and 12, the object to be processed 1 can be an intraocular lens, and the intraocular lens can include a pattern in the haptic part H and the optic part O. The information of the pattern formed, respectively, that is, the shape, depths D1, D2, intervals R1, R2, and widths G1, G2 thereof can be variously formed. That is, although the pattern is extended and formed on the sides of the haptic part H and the optic part O continuously, the pattern information can be variously formed according to the formed location. For example, the intervals between the patterns (R; R1, R2) can be determined in the range of 1 to 15 micrometers, and the widths of the patterns (G; G1, G2) can be determined in the range of 1 to 50 micrometers. Then, the nano surface inside the width of the pattern can be determined in the range of 1 to 800 nanometers. Accordingly, the pattern can be a composite pattern having the width in a micrometer size and the inner surface in a nanometer size. Meanwhile, the pattern information, that is, the shape, depth, interval, width, etc. of the pattern can be variously determined according to the type of cells moving on the pattern. The interval between the patterns or the width of the pattern is described as a case where the three-dimensional object to be processed is an intraocular lens, and the cell moving on the pattern formed on the intraocular lens is an epithelial cell.

In addition, the interval R between the patterns is the same, the width G of the pattern can be variously formed, and in this time, the width G1 of the pattern formed on the optic part O can be formed to be smaller than the width G2 of the pattern formed on the haptic part H.

FIG. 13 is a diagram illustrating a patterning processing sequence in accordance with an embodiment of the present disclosure. Referring to FIG. 13, a processing for sequentially performing the object to be processed A, the three-dimensional profile inspection data B, the data conversion and three-dimensional CAD file C, the pattern file generation and projection D, the three-dimensional pattern data designed on the object to be processed E, outline shape recognition F, alignment of the object to be processed and projection of the pattern data G, and the patterned intraocular lens I is illustrated, and basically, the descriptions explained in FIGS. 4 to 7 can be included therein.

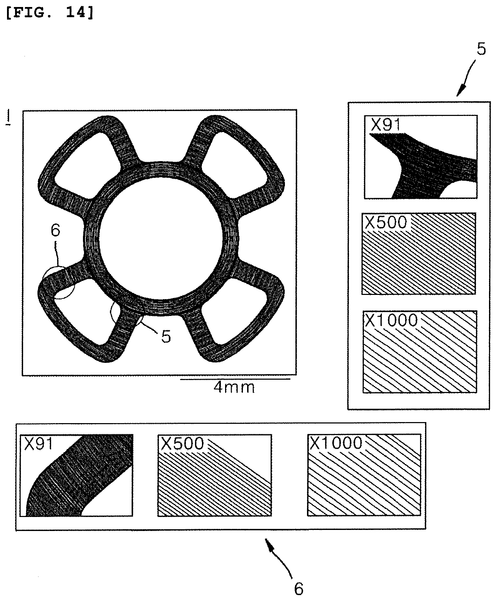

FIG. 14 is a diagram illustrating an intraocular lens in which pattering is completed when the three-dimensional object to be processed is the intraocular lens in accordance with an embodiment of the present disclosure.

Referring to FIG. 14, the three-dimensional transparent object to be processed 1 can be the patterned intraocular lens D manufactured by the processing method described with reference to FIGS. 4 to 7 and FIG. 13. The center of the optic part O is formed so that no pattern is formed thereon and light can pass through without interference of the pattern, and a pattern can be processed on the haptic part H excluding the optic part O and the periphery of the outer circumferential surface of the optic part O. It can be confirmed that a first pattern 5 and a second pattern 6 are magnified by 91 magnifications, 500 magnifications, and 1000 magnifications, respectively.

Meanwhile, the above-described micro-sized pattern can be provided with a predetermined structure. Herein, the predetermined structure can be a nano-sized structure and can include a nano-sized processing projection. In addition, it is natural that the size of the micro pattern and the predetermined structure within the micro-sized pattern can be variously designed and formed according to the type of cell and the function of the pattern. That is, various cells are present in the living body, and the size of the micro pattern and the predetermined structure within the micro-sized pattern can be variously formed according to the type thereof. In addition, depending on the function of the pattern, such as suppressing the movement of the cell or activating the movement of the cell, the micro-sized pattern and the predetermined structure within the micro-sized pattern can be variously formed.

The formation of the processing projection can be formed by outputting the processing laser as the size corresponding to the predetermined structure to be processed on the object to be processed 1. The processing projection can be divided into a side-portion processing projection and a bottom-portion processing projection. The shape and size of the processing projection can be adjusted by laser processing parameters such as a laser output, the overlap rate of laser pulses, and the overlap rate between the patterns. Hereinafter, the case where the three-dimensional object to be processed is an intraocular lens will be described. In addition, in the following description, an epithelial cell is described as an example of the cell, and it is not limited thereto. Actually, it can be various cells in the human body such as a bone cell and an inflammatory cell as well as the epithelial cell in which mobility can be controlled by forming a pattern in various three-dimensional objects to be processed.

When the three-dimensional object to be processed is an intraocular lens and the cell to be controlled is an epithelial cell, the depth of the periphery of the processing projection (the depth between neighboring processing projections) can be formed in the range of 0.1 *µ*m to 30 *µ*m. When the depth is formed to be less than 0.1 *µ*m, the predetermined structure can be difficult to reduce the mobility of the epithelial cell. In addition, the size of 30 *µ*m that is the upper limit of the depth is an example corresponding to the depth of the recess part or the height of the convex part, and is an example of the upper limit value of the predetermined structure provided in the pattern to suppress posterior cataract.

Then, the interval between the processing projections can be formed in the range of 0.1 *µ*m to 10 *µ*m. When the interval between the processing projections is formed to be less than 0.1 *µ*m, the epithelial cell that can cause the posterior cataract can depend on the side portion thereof for its movement, and since the area of the side portion thereof is widened, it is difficult to expect the effect of suppressing the mobility of the cell due to the processing projection. In addition, when the interval between the processing projections exceeds 10 *µ*m, the number of processing projections decreases and thereby, it is difficult to expect the effect of suppressing the mobility of the cell due to the processing projection.

In addition, the width of the processing projection can be formed in the range of 0.1 *µ*m to 10 *µ*m. When the width of the processing projection is less than 0.1 *µ*m or more than 10 *µ*m, it is difficult to achieve the function for suppressing the mobility of the cell due to the processing projection, such that it can be formed in such a size.

The predetermined structure can become a boundary portion thereof. Herein, the boundary portion can mean a structure for controlling the speed and direction in the mobility of the cell moving in the pattern. That is, it means a structure capable of increasing or decreasing the mobility of cell.

A configuration that narrows from the front side to the rear side based on the movement direction of the cell can be formed. Specifically, in the entire width, a cell movement path is formed in which the passing-through cross-sectional area through which the cell is moved is reduced to 1/2 or 1/3, etc., and the movement speed of the cell can be increased. Such a structure can be provided in a part of the pattern described above. Herein, the narrow and wide portions of the passing-through cross-sectional area of the cell can be provided on the front and rear sides based on the movement direction thereof by the predetermined structure, and can be also provided in the opposite direction thereof. That is, it is possible to control the increase or decrease of the mobility of the cell according to the required condition.

The width of the pattern having the highest mobility of the cell and the width of the pattern having the lowest mobility of the cell can be formed in a predetermined section by adjusting the laser processing parameters to control the mobility of the cell.

For example, the pattern having the width and the lowest mobility of the cell can be applied to the section for reducing the movement speed, while the pattern having a narrower width and a higher mobility of the cell can be provided in the section for facilitating the movement speed. As an example, a pattern adjacent to the optic part O can be formed with a pattern having a narrow width and a high mobility.

In addition, the boundary portion, which can be the structure described above, can be a structure that can block the cell from moving in one direction in the pattern and bypass the cell in the other direction therein. The structure of the boundary portion can suppress the movement thereof when the cell moves along the direction of movement.

As a similar structure, referring to FIG. 8(c), the passing-through cross-sectional area at the rear side based on the movement direction Ml of the cell can be reduced to 1/3, for example. At the starting point of the point where the passing-through cross-sectional area of the cell reduces, the boundary portion formed in the oblique direction with respect to the movement direction of the cell can be located. The boundary portion can be extended obliquely from the side portion of the recess portion toward the front side of the movement direction thereof, and this structure can cause the cell to be suppressed or delayed by the boundary portion during the movement thereof. Unlike the case described above, in the opposite direction of movement, the boundary portion can prevent the cell from moving in the opposite direction thereof.

In the opposite direction of movement, the boundary can be applied to the section for reducing the movement speed, and it can be formed at the boundary moved from the optic part O to the haptic part H or at any point on the haptic part H.

As describe above, while the representative embodiments of the present disclosure have been described in detail, those skilled in the art to which the present disclosure pertains will appreciate that various modifications can be made for the above-described embodiment within the scope of the present disclosure without departing from the scope of the present disclosure. Accordingly, the scope of the present disclosure should not be limited to the described embodiments, but should be determined by equivalents to the appended claims, as well as the following claims.

### [Detailed Description of Main Elements]

1: object to be processed
10: laser generation unit
20: first beam adjustment unit
21: beam attenuator
22: beam expander
30: second beam adjustment unit
31: dynamic focusing module
32: scan head
40: mirror unit
50: light collection unit
60: shape recognition unit
70: control unit
S1: loading sample
S1-1: three-dimensional profiling
S2: generating three-dimensional information
S3: generating pattern
S4: laser processing
P1: preparing
P2: forming pattern
P2-1: three-dimensional design
P2-2: scanning sample
P2-3: filtering
P3: processing
P31: laser processing
P32: managing quality
P32a: analysis report
P33: end
H: haptic part
O: optic part
OG: optic groove
HG: haptic groove
D1, D2: depths
R1, R2: intervals
G1, G2: widths
A: object to be processed
B: three-dimensional profile inspection data
C: data conversion and three-dimensional CAD file
D: pattern file generation and projection
E: three-dimensional pattern data designed on the object to be processed
F: outline shape recognition
G: Alignment of the object to be processed and projection of pattern data
I: patterned intraocular lens

## Claims

1. A laser patterning apparatus of a three-dimensional object to be processed, comprising:
a laser generation unit;
a first beam adjustment unit for adjusting the magnitude of a laser beam generated in the laser generation unit;
a second beam adjustment unit for adjusting the focal locations of z-axis, x-axis, and y-axis of the laser beam via the first beam adjustment unit; and
a control unit for controlling the second beam adjustment unit so that laser patterning is performed on a three-dimensional object to be processed,
wherein the control unit is configured to provide information of a pattern to be processed to one of three-dimensional location formation of a loaded three-dimensional object to be processed and three-dimensional location information included in a three-dimensional shape design file, and to perform the alignment through the matching of the three-dimensional location information of the loaded three-dimensional object to be processed and the three-dimensional location information included in the three-dimensional shape design file so that the laser patterning is performed.

2. The laser patterning apparatus of the three-dimensional object to be processed according to claim 1,
wherein the pattern information comprises the shape of the pattern, the width of the pattern, the depth of the pattern, the interval between the patterns, and the wavelength and output, pulse width, scanning speed, spot size, etc. of the laser beam.

3. The laser patterning apparatus of the three-dimensional object to be processed according to claim 1 or 2,
wherein the pattern information is information that applies one of the three-dimensional location information of the loaded three-dimensional object to be processed and the three-dimensional location information included in the three-dimensional shape design file to three-dimensionally convert the shape of the pattern formed on the plane by the control unit.

4. The laser patterning apparatus of the three-dimensional object to be processed according to any one of the preceding claims,
wherein the pattern information is information that generates three-dimensional pattern shape information in one of the three-dimensional shape location information of the loaded three-dimensional object to be processed and the three-dimensional location information included in the three-dimensional shape design file to be converted into three-dimensional information by the control unit.

5. The laser patterning apparatus of the three-dimensional object to be processed according to any one of the preceding claims,
wherein the first beam adjustment unit is a beam expander, and
wherein the second beam adjustment unit comprises a scan head for adjusting the focal locations of the x-axis and the y-axis and a dynamic focusing module for adjusting the focal location of the z-axis.

6. The laser patterning apparatus of the three-dimensional object to be processed according to any one of the preceding claims, comprising a light collection unit for collecting the laser beam on the three-dimensional object to be processed,
wherein the light collection unit comprises a telecentric F-theta lens or an F-theta lens.

7. The laser patterning apparatus of the three-dimensional object to be processed according to claim 5 or 6, insofar as dependent upon claim 5,
wherein the control unit is configured to: extract x-axis, y-axis, and z-axis surface shape data of the three-dimensional object to be processed; and control the dynamic focusing module for adjusting the focal location of the z-axis and the scan head for adjusting the focal locations of the x and y-axes according to the extracted data to form a fine pattern having the pattern width and pattern depth from a micro size to a nano size on the surface of the three-dimensional object to be processed.

8. The laser patterning apparatus of the three-dimensional object to be processed according to any one of the preceding claims, further comprising a shape recognition unit,
wherein the shape recognition unit comprises one of an Optical Coherence Tomography (OCT), a laser interferometer, a confocal microscope, and a two-photon microscope in order to extract surface shape information having x, y, and z-axes of the three-dimensional object to be processed.

9. The laser patterning apparatus of the three-dimensional object to be processed according to any one of the preceding claims,
wherein the three-dimensional object to be processed is a bio-transplantation body, and
wherein the laser beam of one of nanoseconds, picoseconds, or femtoseconds is irradiated to the three-dimensional object to be processed to form a fine pattern.

10. The laser patterning apparatus of the three-dimensional object to be processed according to any one of the preceding claims,
wherein the three-dimensional object to be processed is a bio-transplantation body, and
wherein the laser beam is irradiated to the three-dimensional object to be processed to form a fine pattern, and accordingly, the mobility of a cell moving on the fine pattern is controlled.

11. The laser patterning apparatus of the three-dimensional object to be processed according to claim 10,
wherein one or more of the width, the distance, and the depth of the fine pattern are variously formed according to the type of the cell.

12. A laser patterning method of a three-dimensional object to be processed, comprising:
loading a three-dimensional object to be processed in a laser patterning apparatus;
acquiring an actually measured three-dimensional location information by measuring the surface of the three-dimensional object to be processed;
aligning the three-dimensional object to be processed by matching three-dimensional location information of the object to be processed that has been previously inputted to a control unit on the diagram or the actually measured three-dimensional location information by the control unit; and
processing a pattern by irradiating a laser beam on the aligned three-dimensional object to be processed according to information of the pattern.

13. The laser patterning method of the three-dimensional object to be processed according to claim 12,
wherein the pattern information applies one of the actually measured three-dimensional location information of the loaded three-dimensional object to be processed and three-dimensional location information included in the three-dimensional shape design file to three-dimensionally convert and form the shape of the pattern formed on the plane by the control unit.

14. The laser patterning method of the three-dimensional object to be processed according to claim 12 or 13,
wherein the pattern information is information that directly generates three-dimensional pattern shape information in one of the three-dimensional shape location information of the loaded three-dimensional object to be processed and the three-dimensional location information included in the three-dimensional shape design file to be converted into three-dimensional information by the control unit.

15. The laser patterning method of the three-dimensional object to be processed according to any once of claims 12 to 14, further comprising managing quality for inspecting the shape of the pattern, the width of the pattern, the depth of the pattern, the interval between the patterns, and the wavelength and output, pulse width, scanning speed, and spot size of the laser beam with respect to the object to be processed that the pattern has been processed.
